(19) Europäisches Patentamt
European Patent Office
Office européen des brevets

(11) Veröffentlichungsnummer: **0 039 421**
**B1**

(12) # EUROPÄISCHE PATENTSCHRIFT

(45) Veröffentlichungstag der Patentschrift: **02.11.83**

(51) Int. Cl.³: **C 07 D 249/12,** A 01 N 43/64

(21) Anmeldenummer: **81102698.8**

(22) Anmeldetag: **09.04.81**

(54) **1-(Trihalogenmethyl-sulfenyl)-4-aryl-1,2,4-triazolidin-5-one, Verfahren zu ihrer Herstellung, diese enthaltende Fungizide und Verfahren zur Bekämpfung von Pilzen mit diesen Verbindungen.**

(30) Priorität: **19.04.80 DE 3015090**

(43) Veröffentlichungstag der Anmeldung: **11.11.81 Patentblatt 81/45**

(45) Bekanntmachung des Hinweises auf die Patenterteilung: **02.11.83 Patentblatt 83/44**

(84) Benannte Vertragsstaaten: **AT BE CH DE FR GB IT LI NL SE**

(56) Entgegenhaltungen: **US - A - 4 098 896**

(73) Patentinhaber: **BASF Aktiengesellschaft, Carl-Bosch-Strasse 38, D-6700 Ludwigshafen (DE)**

(72) Erfinder: **Rentzea, Costin, Dr., Neuenheimer Landstrasse 72, D-6900 Heidelberg (DE)**
Erfinder: **Zeeh, Bernd, Dr., Thorwaldsenstrasse 5, D-6700 Ludwigshafen (DE)**
Erfinder: **Pommer, Ernst-Heinrich, Dr., Berliner Platz 7, D-6703 Limburgerhof (DE)**
Erfinder: **Mappes, Celia Joan, Dr., Wiesenweg 145, D-6721 Westheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 039 421 B1

# 1-(Trihalogenmethylsulfenyl)-4-aryl-1,2,4-triazolidin-5-one, Verfahren zu ihrer Herstellung, diese enthaltende Fungizide und Verfahren zur Bekämpfung von Pilzen mit diesen Verbindungen

Die Erfindung betrifft neue wertvolle 1-(Trihalogenmethylsulfenyl)-4-aryl-1,2,4-triazolidin-5-one mit fungizider Wirkung, Verfahren zu ihrer Herstellung sowie deren Verwendung zur Bekämpfung phytophathogener Pilze und Fungizide, die diese Verbindungen enthalten.

Es ist bekannt, N-Trichlormethylthiotetrahydrophthalimid zur Bekämpfung von phytopathogenen Pilzen zu verwenden („Chemical Week", 1972, Juni 21, S. 63). Seine Wirkung ist jedoch gegen andere Phycomyzeten, z.B. *Phytophthora infestans* bei Tomaten oder Kartoffeln, nicht befriedigend. Zum Materialschutz oder zum Schutz von Holz gegen holzverfärbende Pilze genügt seine Wirkung nicht.

Es ist ferner bekannt, 2,4,5,6-Tetrachlorisophthalodinitril zur Bekämpfung von Pilzen zu verwenden („Chemical Week", 1972, Juni 21, S. 55).

Schliesslich ist bekannt, 1-Halomethylthio-3-methylthio-4-phenyl-1,2,4-triazolidin-5-one als Fungizide zu verwenden (US-PS Nr. 4098896). Ihre Wirkung ist jedoch nicht befriedigend.

Es wurde nun gefunden, dass die neuen 1-(Trihalogenmethylsulfenyl-4-aryl-1,2,4-triazolidin-5-one der allgemeinen Formel I

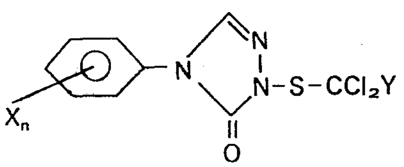

(I)

in der

X Wasserstoff, Halogen (z.B. Fluor, Chlor, Brom oder Jod), eine gegebenenfalls durch Fluor, Chlor oder Brom substituierte Alkyl- oder Alkoxygruppe mit 1 bis 5 C-Atomen, Alkenyl mit 2 bis 4 C-Atomen, Cyan, Nitro, Phenyl oder Phenoxy bedeutet, wobei der Phenyl- und der Phenoxyrest durch Fluor, Chlor, Brom oder Alkyl mit 1 bis 4 C-Atomen substituiert sein kann, und

n eine ganze Zahl von 1 bis 5 ist, und

Y Fluor oder Chlor bedeutet,

eine starke fungizide Wirkung haben. Die neuen Stoffe haben ein breites Wirkungsspektrum und können angewandt werden vor allem gegen Phycomyzeten und *Fungi imperfecti* aber auch gegen Ascomyzeten und Basidiomyzeten. Die neuen Verbindungen sind beispielsweise geeignet zur Anwendung im Pflanzenschutz zur Bekämpfung phytopathogener Pilze, z.B. *Plasmopara viticola* an Reben, *Pseudoperonospora humili* an Hopfen, *Phytophthora infestans* an Kartoffeln und Tomaten, *Pythium ultimum* an Erbsenkeimlingen, *Botrytis cinerea* an Reben, Erdbeeren und Paprika, *Septoria nodorum* an Getreide, *Venturia inaequalis* (Schorf) an Apfelbäumen. Dabei schädigen sie Kulturpflanzen in den zur Bekämpfung von Pilzen notwendigen Konzentrationen nicht. Die neuen 1-(Trihalogenmethylsulfenyl)-4-aryl-1,2,4-triazolidin-5-one eignen sich ferner hervorragend zum Materialschutz und zum Schutz von Holz gegen Pilzarten wie *Sclerophoma* und *Pullullaria*.

Weiterhin wurde gefunden, dass man die Verbindungen der allgemeinen Formel I erhält, wenn man ein 4-Aryl-1,2,4-triazolidin-5-on der allgemeinen Formel II

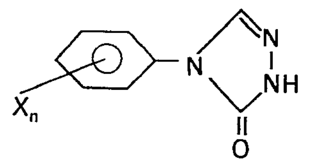

(II)

mit einem Trihalogenmethylsulfenylchlorid der allgemeinen Formel $YCCl_2-S-Cl$ (III), wobei X, Y und n die oben angegebenen Bedeutungen haben, gegebenenfalls in Gegenwart eines Säurebindemittels und gegebenenfalls in Gegenwart eines Lösungs- bzw. Verdünnungsmittels umsetzt. Die erfindungsgemässen Umsetzungen werden zweckmässig in gegenüber den Reaktionsteilnehmern inerten Lösungs- oder Verdünnungsmitteln, z.B. Toluol, Xylol, Diäthyläther, Tetrahydrofuran, Dioxan, Dimethoxyäthan, Aceton, Methyläthylketon, Essigsäureester, Methylenchlorid, Chloroform, Dichloräthan oder Chlorbenzol durchgeführt.

Als säurebindende Mittel kommen z.B. anorganische Basen wie Hydroxide oder Carbonate von Alkali- und Erdalkalimetallen (z.B. NaOH, $NaHCO_3$, $Na_2CO_3$, $KHCO_3$, $K_2CO_3$, $CaCO_3$, $BaCO_3$) und vor allem tertiäre Amine, wie Triäthylamin, N,N-Dimethylcyclohexylamin, N,N-Dimethylanilin oder Pyridin in Frage.

Die Umsetzungen erfolgen beispielsweise bei Temperaturen zwischen −30 und +100° C, vorzugsweise zwischen −10 und +125° C und bei normalem Druck.

Die neuen Verbindungen der allgemeinen Formel I, wobei Y Fluor bedeutet, erhält man ferner, wenn man ein 1,2,4-Triazolidin-5-on der allgemeinen Formel Ia

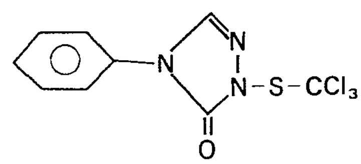

(Ia)

in der X und n die oben angegebenen Bedeutungen haben, mit wasserfreier Fluorwasserstoffsäure umsetzt, um in der Trichlormethylthio-Seitenkette ein Chloratom gegen Fluor auszutauschen. Die erfindungsgemässe Umsetzung mit einer Verbindung der Formel Ia kann in einem Überschuss von Fluorwasserstoffsäure als Verdünnungsmittel bei Temperaturen zwischen −50 und +80° C, vorzugsweise zwischen −10 und +25° C unter normalem oder erhöhtem Druck durchgeführt werden.

Die als Ausgangsstoffe zu verwendenden 4-Aryl-1,2,4-triazolidin-5-one sind durch die allgemeine Formel II allgemein definiert. In dieser Formel steht X vorzugsweise für Wasserstoff, Fluor, Chlor, Brom, Methyl, Äthyl, Isopropyl, tert.-Butyl, Trifluormethyl, Nitro, Methoxy, Äthoxy, Tetrafluoräthoxy, Phenoxy und Phenyl, wobei n vorzugsweise für 1, 2, 3 oder 4 steht.

Die Ausgangsstoffe der allgemeinen Formel II sind bekannt [vgl. H. Gehlen u. W. Schade, „Liebigs Ann. Chem.", *675*, 180 (1964) und M. Pesson u. S. Dupin, „Bull. Soc. Chim., France", 250 (1962)]. Noch nicht bekannte Ausgangsstoffe der allgemeinen Formel II können nach dem bekannten Verfahren erhalten werden, indem man 1-Aryl-4-formylsemicarbazide in Gegenwart von Alkalihydroxiden cyclisiert.

Die weiterhin für die Herstellung der erfindungsgemässen Stoffe erforderlichen 1-Trichlormethylsulfenyl-4-aryl-1,2,4-triazolidin-3-one sind durch die allgemeine Formel Ia allgemein definiert. Sie können nach prinzipiell bekannten und laboratoriumsüblichen Verfahren hergestellt werden. Angaben dazu finden sich bei den Herstellungsbeispielen.

Die schliesslich für die Herstellung der erfindungsgemässen Stoffe erforderlichen Trihalogenmethylsulfenylchloride der allgemeinen Formel III sind allgemein bekannt.

Als Beispiele für die neuen Wirkstoffe seien im einzelnen genannt:

1-Trichlormethylsulfenyl-4-phenyl-1,2,4-triazolidin-5-on,
1-Fluordichlormethylsulfenyl-4-phenyl-1,2,4-triazolidin-5-on,
1-Trichlormethylsulfenyl-4-(2-chlorphenyl)-1,2,4-triazolidin-5-on,
1-Fluordichlormethylsulfenyl-4-(2-chlorphenyl)-1,2,4-triazolidin-5-on,
1-Trichlormethylsulfenyl-4-(3-chlorphenyl)-1,2,4-triazolidin-5-on,
1-Fluordichlormethylsulfenyl-4-(3-chlorphenyl)-1,2,4-triazolidin-5-on,
1-Trichlormethylsulfenyl-4-(4-chlorphenyl)-1,2,4-triazolidin-5-on,
1-Fluordichlormethylsulfenyl-4-(4-chlorphenyl)-1,2,4-triazolidin-5-on,
1-Trichlormethylsulfenyl-4-(2,4-dichlorphenyl)-1,2,4-triazolidin-5-on,
1-Fluordichlormethylsulfenyl-4-(2,4-dichlorphenyl)-1,2,4-triazolidin-5-on,
1-Trichlormethylsulfenyl-4-(3,4-dichlorphenyl)-1,2,4-triazolidin-5-on,
1-Fluordichlormethylsulfenyl-4-(3,4-dichlorphenyl)-1,2,4-triazolidin-5-on,
1-Trichlormethylsulfenyl-4-(3,5-dichlorphenyl)-1,2,4-triazolidin-5-on,
1-Fluordichlormethylsulfenyl-4-(3,5-dichlorphenyl)-1,2,4-triazolidin-5-on,
1-Trichlormethylsulfenyl-4-(2,4,5-trichlorphenyl)-1,2,4-triazolidin-5-on,
1-Trichlormethylsulfenyl-4-(4-fluorphenyl)-1,2,4-triazolidin-5-on,
1-Fluordichlormethylsulfenyl-4-(4-fluorphenyl)-1,2,4-triazolidin-5-on,
1-Trichlormethylsulfenyl-4-(3,4-difluorphenyl)-1,2,4-triazolidin-5-on,
1-Trichlormethylsulfenyl-4-(4-bromphenyl)-1,2,4-triazolidin-5-on,
1-Trichlormethylsulfenyl-4-(4-methylphenyl)-1,2,4-triazolidin-5-on,
1-Fluordichlormethylsulfenyl-4-(4-methylphenyl)-1,2,4-triazolidin-5-on,
1-Trichlormethylsulfenyl-4-(2,4-dimethylphenyl)-1,2,4-triazolidin-5-on,
1-Trichlormethylsulfenyl-4-(2-methyl-4-chlorphenyl)-1,2,4-triazolidin-5-on,
1-Fluordichlormethylsulfenyl-4-(2-methyl-4-chlorphenyl)-1,2,4-triazolidin-5-on,
1-Trichlormethylsulfenyl-4-(3-trifluormethylphenyl)-1,2,4-triazolidin-5-on,
1-Fluordichlormethylsulfenyl-4-(3-trifluormethylphenyl)-1,2,4-triazolidin-5-on,
1-Trichlormethylsulfenyl-4-(4-trifluormethylphenyl)-1,2,4-triazolidin-5-on,
1-Trichlormethylsulfenyl-4-(3-nitrophenyl)-1,2,4-triazolidin-5-on,
1-Fluordichlormethylsulfenyl-4-(3-nitrophenyl)-1,2,4-triazolidin-5-on,
1-Trichlormethylsulfenyl-4-(4-nitrophenyl)-1,2,4-triazolidin-5-on,
1-Fluordichlormethylsulfenyl-4-(4-nitrophenyl)-1,2,4-triazolidin-5-on,
1-Trichlormethylsulfenyl-4-(4-isopropylphenyl)-1,2,4-triazolidin-5-on,
1-Fluordichlormethylsulfenyl-4-(4-isopropylphenyl)-1,2,4-triazolidin-5-on,
1-Trichlormethylsulfenyl-4-(4-tert.-butylphenyl)-1,2,4-triazolidin-5-on,
1-Trichlormethylsulfenyl-4-(3-tert.-butylphenyl)-1,2,4-triazolidin-5-on,
1-Fluordichlormethylsulfenyl-4-(3-tert.-butylphenyl)-1,2,4-triazolidin-5-on,
1-Trichlormethylsulfenyl-4-(4-methoxyphenyl)-1,2,4-triazolidin-5-on,
1-Fluordichlormethylsulfenyl-4-(4-methoxyphenyl)-1,2,4-triazolidin-5-on,
1-Trichlormethylsulfenyl-4-(3-chlor-4-methoxyphenyl)-1,2,4-triazolidin-5-on,
1-Fluordichlormethylsulfenyl-4-(3-chlor-4-methoxyphenyl)-1,2,4-triazolidin-5-on,
1-Trifluormethylsulfenyl-4-(3,5-dichlor-4-methoxyphenyl)-1,2,4-triazolidin-5-on,
1-Fluordichlormethylsulfenyl-4-(3,5-dichlor-4-methoxyphenyl)-1,2,4-triazolidin-5-on,
1-Trichlormethylsulfenyl-4-(3-tetrafluoräthoxyphenyl)-1,2,4-triazolidin-5-on,
1-Trichlormethylsulfenyl-4-(4-tetrafluoräthoxyphenyl)-1,2,4-triazolidin-5-on,
1-Fluordichlormethylsulfenyl-4-(3-tetrafluoräthoxyphenyl)-1,2,4-triazolidin-5-on,
1-Fluordichlormethylsulfenyl-4-(4-tetrafluoräthoxyphenyl-1,2,4-triazolidin-5-on,
1-Trichlormethylsulfenyl-4-[4-(4-chlorphenoxy)phenyl]-1,2,4-triazolidin-5-on,
1-Fluordichlormethylsulfenyl-4-[4-(4-chlorphenoxy)phenyl]-1,2,4-triazolidin-5-on,
1-Trichlormethylsulfenyl-4-(4-bisphenylyl)-1,2,4-triazolidin-5-on,
1-Fluordichlormethylsulfenyl-4-(4-bisphenylyl)-1,2,4-triazolidin-5-on.

Die folgenden Versuchsangaben erläutern die Herstellung der Wirkstoffe.

*Herstellungsbeispiele*

*Beispiel 1*

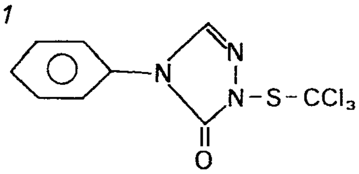

Zu einer Suspension von 16,1 g (0,1 mol) 4-Phenyl-1,2,4-triazolidin-5-on in 150 ml trokkenem Essigester werden bei etwa 10 bis 15° C nacheinander 19 g (0,102 mol) Perchlormethylmercaptan und 10 g (0,099 mol) Triäthylamin unter gutem Rühren zugetropft. Nach zweistündigem Rühren bei Raumtemperatur (20° C) wird das ausgefallene Triäthylaminhydrochlorid abgesaugt und mit 40 ml Essigester gewaschen. Das Filtrat wird zweimal mit je 100 ml Wasser gewaschen (ausgeschüttelt), über $Na_2SO_4$ getrocknet und das Lösungsmittel verdampft (eingeengt). Der Rückstand kristallisiert bei 0° C nach Zugabe von 20 ml Äther.

Man erhält 25,8 g (83% d. Th.) 1-Trichlormethylsulfenyl-4-phenyl-1,2,4-triazolidin-5-on als weisse Kristalle vom Schmelzpunkt 169 bis 171° C (Nr. 1).

*Beispiel 2*

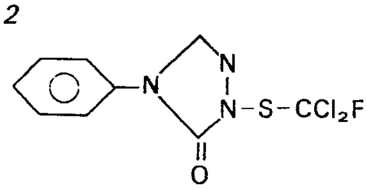

Entsprechend Beispiel 1 werden 16,1 g (0,1 mol) 4-Phenyl-1,2,4-triazolidin-5-on mit 17 g (0,1 mol) Fluordichlormethylsulfenylchlorid umgesetzt. Man erhält 20,9 g (76% d. Th.) 1-Fluordichlormethylsulfenyl-4-phenyl-1,2,4-triazolidin-5-on als weisse Kristalle vom Schmelzpunkt 128 bis 130° C (Nr. 2).

Analog wurden die folgenden Verbindungen der allgemein Formel I hergestellt, die durch Ultrarot- und Kernresonanz-Spektroskopie sowie durch Elementaranalyse charakterisiert wurden.

| Beispiel Nr. | X | X | Schmelzpunkt (°C) |
|---|---|---|---|
| 3 | 4-Cl | Cl | 174-178 |
| 4 | 4-Cl | F | 151-153 |
| 5 | 3-Cl | Cl | 155-158 |
| 6 | 3-Cl | F | 118-120 |
| 7 | 3,5-$Cl_2$ | Cl | 187-190 |
| 8 | 4-F | Cl | 145-147 |
| 9 | 4-F | F | 113-115 |
| 10 | 3-$F_3C$– | Cl | 150-153 |
| 11 | 3-$F_3C$– | F | 111-116 |
| 12 | 2-$CH_3$–, 4-Cl | Cl | 84-87 |
| 13 | 2-$CH_3$, 4-Cl | F | Harz |
| 14 | 3-Cl, 4-$OCH_3$ | Cl | 198-200 |
| 15 | 3-Cl, 4-$OCH_3$ | F | 152-155 |
| 16 | 4-$OCH_3$ | Cl | 161-163 |
| 17 | 3-tert.-$C_4H_9$ | Cl | 163-165 |
| 18 | 3-tert.-$C_4H_9$ | F | 142-144 |
| 19 | 4-$OCH_3$ | F | 114-116 |

Die neuen Verbindungen zeichnen sich durch eine hervorragende Wirksamkeit gegen ein breites Spektrum von pflanzenpathogenen Pilzen aus. Sie können als Blatt- und Bodenfungizide eingesetzt werden.

Die neuen Verbindungen sind insbesondere geeignet zur Bekämpfung folgender Pflanzenkrankheiten:

*Phytophthora infestans* an Tomaten und Kartoffeln,
*Phytophthora parasitica* an Erdbeeren,
*Phytophthora cactorum* an Äpfeln,
*Pseudoperonospora cubensis* an Gurken,
*Pseudoperonospora humili* an Hopfen,
*Peronospora destructor* an Zwiebeln,
*Peronospora sparsa* an Rosen,
*Peronospora tabacina* an Tabak,
*Plasmopara viticola* an Reben,
*Plasmopara halstedii* an Sonnenblumen,
*Pythium ultimum* an Erbsenkeimlingen,
*Botrytis cinerea* an Reben, Erdbeeren und Paprika,
*Septoria nodorum* an Getreide,
*Venturia inaequalis* (Schorf) an Apfelbäumen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die erfindungsgemässen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollten in jedem Fall eine feine und gleichmässige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Äthanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene

und anionische Emulgatoren (z.B. Polyoxyäthylenfettalkoholäther, Alkylsulfonate und Arylsulfonate) und Dispergiermittel wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,1 und 3 kg Wirkstoff oder mehr je Hektar. Die neuen Verbindungen können auch in Materialschutz eingesetzt werden. Bei der Anwendung der Wirkstoffe im Materialschutz, z.B. als Fungizide für Anstrichfarben und Weichpolyvinylchlorid, betragen die Aufwandmengen 0,05 bis 5% (Gew.-%) Wirkstoff, bezogen auf das Gesamtgewicht der zu konservierenden Farben bzw. des mikrozid auszurüstenden Polyvinylchlorids. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, dass man das Holz mit diesen Mitteln behandelt, beispielsweise tränkt oder anstreicht.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Giessen.

Beispiele für solche Zubereitungen sind:

I. Man vermischt 90 Gew.-Teile der Verbindung des Beispiels 1 mit 10 Gew.-Teilen N-Methyl-α-pyrrolidon und erhält eine Lösung, die zur Anwendung in Form kleinster Tropfen geeignet ist.

II. 10 Gew.-Teile der Verbindung des Beispiels 2 werden in einer Mischung gelöst, die aus 90 Gew.-Teilen Xylol, 6 Gew.-Teilen des Anlagerungsproduktes von 8 bis 10 mol Ölsäure-N-monoäthanolamid, 2 Gew.-Teilen Calciumsalz der Dodecylbenzolsulfonsäure und 2 Gew.-Teilen des Anlagerungsproduktes von 40 mol Äthylenoxid an 1 mol Rizinusöl besteht.

III. 20 Gew.-Teile der Verbindung des Beispiels 3 werden in einer Mischung gelöst, die aus 60 Gew.-Teilen Cyclohexanon, 30 Gew.-Teilen Isobutanol, 10 Gew.-Teilen des Anlagerungsproduktes von 40 mol Äthylenoxid an 1 mol Rizinusöl besteht. Durch Eingiessen und feines Verteilen der Lösung in 100000 Gew.-Teilen Wasser erhält man eine wässerige Dispersion, die 0,02 Gew.-% des Wirkstoffs enthält.

IV. 20 Gew.-Teile der Verbindung des Beispiels 5 werden in einer Mischung gelöst, die aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 mol Äthylenoxid an 1 mol Rizinusöl besteht. Durch Eingiessen und feines Verteilen der Lösung in 100000 Gew.-Teilen Wasser erhält man eine wässerige Dispersion, die 0,02 Gew.-% des Wirkstoffs enthält.

V. 80 Gew.-Teile der Verbindung des Beispiels 2 werden mit 3 Gew.-Teilen des Natriumsalzes des Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

VI. 3 Gew.-Teile der Verbindung des Beispiels 6 werden mit 97 Gew.-Teilen feinteiligem Kaolin innig vermischt. Man erhält auf diese Weise ein Stäubemittel, das 3 Gew.-% des Wirkstoffs enthält.

VII. 30 Gew.-Teile der Verbindung des Beispiels 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung des Beispiels 3 werden mit 10 Teilen Natriumsalz eines Phenolsulfonsäureharnstoffformaldehydkondensates, 2 Teilen Kieselgel und 48 Teilen Wasser innig vermischt. Man erhält eine stabile wässerige Dispersion. Durch Verdünnen mit 100000 Gew.-Teilen Wasser erhält man eine wässerige Dispersion, die 0,04 Gew.-% Wirkstoff enthält.

IX. 20 Teile der Verbindung des Beispiels 1 werden mit 2 Teilen Calciumsalz der Dodecylbenzolsulfonsäure, 8 Teilen Fettalkoholpolyglykoläther, 2 Teilen Natriumsalz eines Phenolsulfonsäureharnstofformaldehydkondensates und 68 Teilen eines paraffinischen Mineralöls innig vermischt. Man erhält eine stabile ölige Dispersion.

Die erfindungsgemässen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrösserung des fungiziden Wirkungsspektrums.

Die folgende Liste von Fungiziden, mit denen die erfindungsgemässen Verbindungen kombiniert werden können, soll die Kombinationsmöglichkeiten erläutern, nicht aber einschränken.

Fungizide, die mit den erfindungsgemässen Verbindungen kombiniert werden können, sind beispielsweise:

Schwefel,
Dithiocarbamate und deren Derivate, wie
Ferridimethyldithiocarbamat,
Zinkdimethyldithiocarbamat,
Manganzinkäthylendiaminbisdithiocarbamat, und
Zinkäthylenbisdithiocarbamat,
Tetramethylthiuramdisulfide,
Ammoniakkomplex von Zink-(N,N-äthylenbis-dithiocarbamat), und
N,N'-Polyäthylenbis(thiocarbamoyl)disulfid,
Zink-(N,N'-propylenbisdithiocarbamat),
Ammoniakkomplex von Zink-(N,N'-propylenbisdithiocarbamat), und

N,N'-Polypropylenbis(thiocarbamoyl)disulfid;
Nitroderivate, wie
Dinitro-(1-methylheptyl)phenylcrotonat,
2-sek.-Butyl-4,6-dinitrophenyl-3,3-dimethylacrylat,
2-sek.-Butyl-4,6-dinitrophenylisopropylcarbonat;
heterocyclische Substanzen, wie
N-(1,1,2,2,-Tetrachloräthylthio)tetrahydrophthalimid,
N-Trichlormethylthiotetrahydrophthalimid,
2-Heptadecyl-2-imidazolinacetat,
2,4-Dichlor-6-(o-chloranilino)-s-triazin,
O,O-Diäthylphthalimidophosphonothioat,
5-Amino-1-[bis(dimethylamino)phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazolcarbaminsäuremethylester,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-[Furyl-(2)]-benzimidazol,
Piperazin-1,4-diylbis-[1-(2,2,2-trichloräthyl)]formamid,
2-Thiazolyl-(4)-benzimidazol,
5-Butyl-2-dimehtylamiano-4-hydroxy-6-methylpyrimidin,
Bis-(p-chlorphenyl)-3-pyridinmethanol,
1,2-Bis-(3-äthoxycarbonyl-2-thioureido)-benzol,
1,2-Bis-(3-methoxycarbonyl-2-thioureido)-benzol

sowie verschiedene Fungizide, wie

Dodecylguanidinacetat,
3-[3-(3,5-Dimethyl-2-oxycyclohexyl)-2-hydroxyäthyl]glutarimid,
Hexachlorbenzol,
N-Dichlorfluormethylthio-N',N'-dimethyl-N-phenylschwefelsäurediamid,
2,5-Dimethylfuran-3-carbonsäureanilid,
2-Methylbenzoesäureanilid,
2-Jodbenzoesäureanilid,
1-(3,4-Dichloranilino)-1-formylamino-2,2,2-trichloräthan,
2,6-Dimethyl-N-tridecylmorpholin bzw. dessen Salze,
2,6-Dimethyl-N-cyclododecylmorpholin bzw. dessen Salze,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidinmethanol.

Für die folgenden Versuche wurden die folgenden bekannten Wirkstoffe als Vergleichsmittel verwendet:

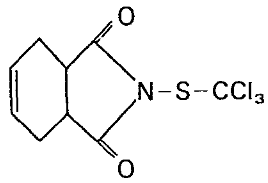

Vergleichsmittel A

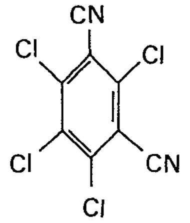

Vergleichsmittel B

*Versuch 1*

*Wirksamkeit gegen* Phytophthora infestans *an Tomaten*

Blätter von Topfpflanzen der Sorte Grosse Fleischtomate werden mit wässerigen Suspensionen, die 0,025% (Gew.-%) Wirkstoff enthalten, besprüht. Nach dem Antrocknen des Spritzbelages werden die Blätter mit einer Zoosporenaufschwemmung des Pilzes *Phytophthora infestans* infiziert. Die Pflanzen werden dann in einer wasserdampfgesättigten Kammer bei Temperaturen zwischen 16 und 18° C aufgestellt. Nach 5 d hat sich die Krankheit auf den unbehandelten, jedoch infizierten Kontrollpflanzen so stark entwickelt, dass die fungizide Wirksamkeit der Substanzen beurteilt werden kann.

Die neuen Verbindungen 1, 3, 4, 5, 6, 7 zeigten eine bessere fungizide Wirksamkeit als die bekannte Verbindung A.

*Versuch 2*

Septoria nodorum *an Weizen*

Blätter von in Töpfen gewachsenen Weizenpflanzen der Sorte Jubilar werden mit wässerigen Spritzbrühen, die 0,05 und 0,1% (Gew.-%) Wirkstoff emulgiert enthalten, tropfnass gespritzt. Nach dem Antrocknen des Spritzbelages werden die Pflanzen mit einer Sporenaufschwemmung des Pilzes *Septoria nodorum* besprüht und bei 16 bis 18° C in eine Kammer mit hoher Luftfeuchtigkeit gestellt, um optimale Bedingungen für die Pilzentwicklung zu erhalten. Nach 14 d hat sich die Krankheit auf den unbehandelten Kontrollpflanzen so stark entwickelt, dass die entstandenen Blattflecken den überwiegenden Teil der Blätter bedecken.

Die neuen Verbindungen 1, 5, 6 zeigten eine bessere fungizide Wirksamkeit als die bekannte Verbindung B.

**Patentansprüche für die Vertragsstaaten: BE, CH, DE, FR, GB, IT, LI, NL, SE**

1. 1-(Trihalogenmethylsulfenyl)-4-aryl-1,2,4-triazolidin-5-on der allgemeinen Formel (I)

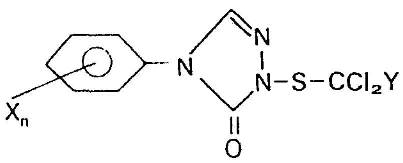 (I)

in der

X Wasserstoff, Halogen, eine gegebenenfalls durch Fluor, Chlor oder Brom substituierte Alkyl- oder Alkoxygruppe mit 1 bis 5 C-Atomen, Alkenyl mit 2 bis 4 C-Atomen, Cyan, Nitro, Phenyl oder Phenoxy bedeutet, wobei der Phenyl- und der Phenoxyrest durch Fluor, Chlor, Brom oder Alkyl mit 1 bis 4 C-Atomen substituiert sein kann, und

n eine ganze Zahl von 1 bis 5 ist, und

Y Fluor oder Chlor bedeutet.

2. Verbindung gemäss Anspruch 1, dadurch gekennzeichnet, dass X Wasserstoff, Chlor, Fluor, Trifluormethyl, Methyl, Butyl oder Methoxy, und n 1 oder 2 bedeutet.

3. Verfahren zur Herstellung eines 1-(Trihalogenmethylsulfenyl)-4-aryl-1,2,4-triazolidin-5-ons gemäss allgemeiner Formel (I) in Anspruch 1, dadurch gekennzeichnet, dass man ein 4-Aryl-1,2,4-triazolidin-5-on der allgemeinen Formel (II)

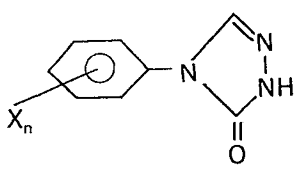 (II)

worin X und n die in Anspruch 1 angegebene Bedeutung haben, mit einem Trihalogenmethylsulfenylchlorid der allgemeinen Formel (III)

$$YCCL_2-S-Cl \qquad (III)$$

worin Y die in Anspruch 1 angegebene Bedeutung hat, gegebenenfalls in Gegenwart eines Lösungsmittels, gegebenenfalls unter Zusatz einer anorganischen oder organischen Base bei Temperaturen zwischen −30 und +100° C umsetzt.

4. Fungizid, enthaltend ein 1-(Trihalogenmethylsulfenyl)-4-aryl-1,2,4-triazolidin-5-on gemäss allgemeiner Formel (I) in Anspruch 1.

5. Fungizid gemäss Anspruch 4, dadurch gekennzeichnet, dass X Wasserstoff, Chlor, Fluor, Trifluormethyl, Methyl, Butyl oder Methoxy, und n 1 oder 3 bedeutet.

6. Fungizid, enthaltend einen üblichen festen oder flüssigen Trägerstoff und ein 1-(Trihalogenmethylsulfenyl)-4-aryl-1,2,4-triazolidin-5-on gemäss allgemeiner Formel (I) in Anspruch 1.

7. Verfahren zur Herstellung eines Fungizids nach Anspruch 6, dadurch gekennzeichnet, dass man einen üblichen festen oder flüssigen Trägerstoff vermischt mit einem 1-(Trihalogenmethylsulfenyl)-4-aryl-1,2,4-triazolidin-5-on gemäss allgemeiner Formel (I) in Anspruch 1.

8. Verfahren zur Bekämpfung von Pilzen ausser humanpathogenen Pilzen, dadurch gekennzeichnet, dass man die Pilze oder die vor Pilzbefall zu schützenden Gegenstände behandelt mit einem 1-(Trihalogenmethylsulfenyl)-4-aryl-1,2,4-triazolidin-5-on gemäss allgemeiner Formel (I) in Anspruch 1.

**Patentansprüche für der Vertragsstaat: AT**

1. Fungizid, enthaltend ein 1-(Trihalogenmethylsulfenyl)-4-aryl-1,2,4-triazolidin-5-on der allgemeinen Formel (I)

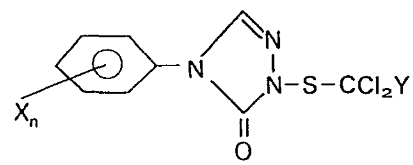 (I)

in der

X Wasserstoff, Halogen, eine gegebenenfalls durch Fluor, Chlor oder Brom substituierte Alkyl- oder Alkoxygruppe mit 1 bis 5 C-Atomen, Alkenyl mit 2 bis 4 C-Atomen, Cyan, Nitro, Phenyl oder Phenoxy bedeutet, wobei der Phenyl- und der Phenoxyrest durch Fluor, Chlor, Brom oder Alkyl mit 1 bis 4 C-Atomen substituiert sein kann, und

n eine ganze Zahl von 1 bis 5 ist, und

Y Fluor oder Chlor bedeutet.

2. Fungizid, enthaltend einen üblichen festen oder flüssigen Trägerstoff und ein 1-(Trihalogenmethylsulfenyl)-4-aryl-1,2,4-triazolidin-5-on gemäss allgemeiner Formel (I) in Anspruch 1.

3. Fungizid gemäss Anspruch 1, dadurch gekennzeichnet, dass X Wasserstoff, Chlor, Fluor, Trifluormethyl, Methyl, Butyl oder Methoxy, und n 1 oder 2 bedeutet.

4. Verfahren zur Herstellung eines Fungizids nach Anspruch 2, dadurch gekennzeichnet, dass man einen üblichen festen oder flüssigen Trägerstoff vermischt mit einem 1-(Trihalogenmethylsulfenyl)-4-aryl-1,2,4-triazolidin-5-on gemäss allgemeiner Formel (I) Anspruch 1.

5. Verfahren zur Bekämpfung von Pilzen ausser humanpathogenen Pilzen, dadurch gekennzeichnet, dass man die Pilze oder die vor Pilzbefall zu schützenden Gegenstände behandelt mit einem 1-(Trihalogenmethylsulfenyl)-4-aryl-1,2,4-triazolidin-5-on gemäss allgemeiner Formel (I) in Anspruch 1.

6. Verfahren zur Herstellung eines 1-(Trihalogenmethylsulfenyl)-4-aryl-1,2,4-triazolidin-5-ons gemäss allgemeiner Formel (I) in Anspruch 1, dadurch gekennzeichnet, dass man ein 4-Aryl-1,2,4-triazolidin-5-on der allgemeinen Formel (II)

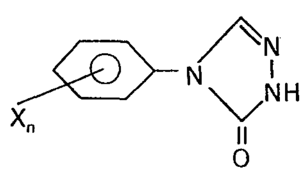 (II)

worin X und n die in Anspruch 1 angegebene Bedeutung haben, mit einem Trihalogenmethylsulfenylchlorid der allgemeinen Formel (III)

$$YCCl_2-S-Cl \qquad (III)$$

worin Y die in Anspruch 1 angegebene Bedeutung hat, gegebenenfalls in Gegenwart eines

Lösungsmittels, gegebenenfalls unter Zusatz einer anorganischen oder organischen Base bei Temperaturen zwischen −30 und +100° C umsetzt.

**Claims for the Contracting States: BE, CH, DE, FR, GB, IT, LI, NL, SE**

1. A 1-(trihalomethyl-sulfenyl)-4-aryl-1,2,4-triazolidin-5-one of the general formula (I)

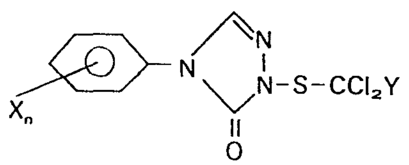 (I)

where

X is hydrogen, halogen, alkyl or alkoxy of 1 to 5 carbon atoms which are unsubstituted or substituted by fluorine, chlorine or bromine, alkenyl of 2 to 4 carbon atoms, cyano or nitro, or phenyl or phenoxy which are unsubstituted or substituted by fluorine, chlorine, bromine or alkyl of 1 to 4 carbon atoms,

n is an integer from 1 to 5, and

Y is fluorine or chlorine.

2. A compound as claimed in claim 1, wherein X is hydrogen, chlorine, fluorine, trifluoromethyl, methyl, butyl or methoxy, and n is 1 or 2.

3. A process for the preparation of a 1-(trihalomethyl-sulfenyl)-4-aryl-1,2,4-triazolidin-5-one of the general formula (I) given in claim 1, wherein a 4-aryl-1,2,4-triazolidin-5-one of the general formula (II)

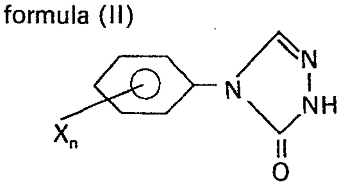 (II)

where X and n have the meanings given in claim 1, is reacted, at a temperature of from −30 to +100° C, with a trihalomethyl-sulfenyl chloride of the general formula (III)

$$YCCl_2-S-Cl \qquad (III)$$

where Y has the meaning given in claim 1, in the presence or absence of a solvent and in the presence or absence of an inorganic or organic base.

4. A fungicide containing a 1-(trihalomethyl-sulfenyl)-4-aryl-1,2,4-triazolidin-5-one of the general formula (I) given in claim 1.

5. A fungicide as claimed in claim 4, wherein X is hydrogen, chlorine, fluorine, trifluoromethyl, methyl, butyl or methoxy, and n is 1 or 2.

6. A fungicide containing a conventional solid or liquid carrier and a 1-(trihalomethyl-sulfenyl)-4-aryl-1,2,4-triazolidin-5-one of the general formula (I) given in claim 1.

7. A process for the production of a fungicide as claimed in claim 6, wherein a conventional solid or liquid carrier is mixed with a 1-(trihalomethyl-sulfenyl)-4-aryl-1,2,4-triazolidin-5-one of the general formula (I) given in claim 1.

8. A method of controlling fungi except fungi which are pathogenic to humans, wherein the fungi or the objects to be protected from fungal attack are treated with a 1-(trihalomethyl-sulfenyl)-4-aryl-1,2,4-triazolidin-5-one of the general formula (I) given in claim 1.

**Claims for the Contracting State: AT**

1. A fungicide containing a 1-(trihalomethyl-sulfenyl)-4-aryl-1,2,4-triazolidin-5-one of the general formula (I)

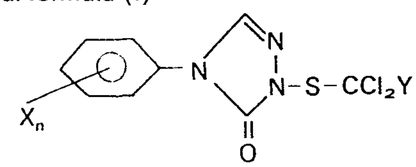 (I)

where

X is hydrogen, halogen, alkyl or alkoxy of 1 to 5 carbon atoms which are unsubstituted or substituted by fluorine, chlorine or bromine, alkenyl of 2 to 4 carbon atoms, cyano or nitro, or phenyl or phenoxy which are unsubstituted or substituted by fluorine, chlorine, bromine or alkyl of 1 to 4 carbon atoms,

n is an integer from 1 to 5, and

Y is fluorine or chlorine.

2. A fungicide containing a conventional solid or liquid carrier and a 1-(trihalomethyl-sulfenyl)-4-aryl-1,2,4-triazolidin-5-one of the general formula (I) given in claim 1.

3. A fungicide as claimed in claim 1, wherein X is hydrogen, chlorine, fluorine, trifluoromethyl, methyl, butyl or methoxy, and n is 1 or 2.

4. A process for the production of a fungicide as claimed in claim 2, wherein a conventional solid or liquid carrier is mixed with a 1-(trihalomethyl-sulfenyl)-4-aryl-1,2,4-triazolidin-5-one of the general formula (I) given in claim 1.

5. A method of controlling fungi except fungi which are pathogenic to humans, wherein the fungi or the objects to be protected from fungal attack are treated with a 1-(trihalomethyl-sulfenyl)-4-aryl-1,2,4-triazolidin-5-one of the general formula (I) given in claim 1.

6. A process for the preparation of a 1-(trihalomethyl-sulfenyl)-4-aryl-1,2,4-triazolidin-5-one of the general formula (I) given in claim 1, wherein a 4-aryl-1,2,4-triazolidin-5-one of the general formula (II)

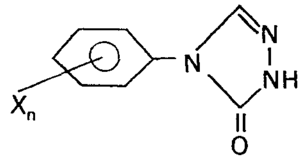 (II)

where X and n have the meanings given in claim 1, is reacted, at a temperature of from −30 to +100° C, with a trihalomethyl-sulfenyl chloride of the general formule (III)

$$YCCl_2-S-Cl \qquad (III)$$

where Y has the meaning given in claim 1, in the presence or absence of a solvent and in the presence or absence of an inorganic or organic base.

**Revendications pour les Etats contractants: BE, CH, DE, FR, GB, IT, NL, LI, SE**

1. 1-(Trihalogénométhylsulfényl)-4-aryl-1,2,4-triazolidin-5-one de formule (I)

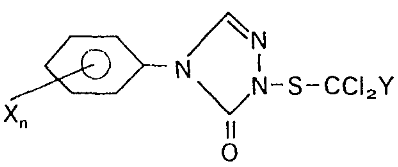

(I)

dans laquelle

X représente hydrogène, halogène, un groupe alkyle ou alcoxy ayant 1 à 5 atomes C, éventuellement substitué par fluor, chlore ou brome, alcényle ayant 2 à 4 atomes C, cyano, nitro, phényle ou phénoxy, le reste phényle ou phénoxy pouvant être substitué par fluor, chlore, brome ou alkyle ayant 1 à 4 atomes C, et

n est un nombre entier de 1 à 5, et

Y représente fluor ou chlore.

2. Composé selon la revendication 1, caractérisé par le fait que X représente hydrogène, chlore, fluor, trifluorométhyle, méthyle, butyle ou méthoxy, et n est 1 ou 2.

3. Procédé de préparation d'une 1-(trihalogénométhylsulfényl)-4-aryl-1,2,4-triazolidin-5-one selon la formule générale (I) de la revendication 1, caractérisé par le fait qu'on fait réagir, éventuellement en présence d'un solvant, éventuellement avec addition d'une base inorganique ou organique, à des températures comprises entre −30 et +100° C, une 4-aryl-1,2,4-triazolidin-5-one de formule générale (II)

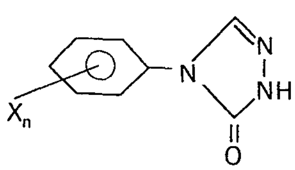

(II)

dans laquelle X et n ont la signification donnée dans la revendication 1, avec un sulfénylchlorure de trihalogénométhyle de formule générale (III)

$$YCCl_2-S-Cl \qquad (III)$$

dans laquelle Y a la signification donnée dans la revendication 1.

4. Fongicide, contenant une 1-(trihalogénométhylsulfényl)-4-aryl-1,2,4-triazolidin-5-one selon la formule générale (I) de la revendication 1.

5. Fongicide selon la revendication 4, caractérisé par le fait que X représente hydrogène, chlore, fluor, trifluorométhyle, méthyle, butyle ou méthoxy, et n est 1 ou 2.

6. Fongicide, contenant un véhicule solide ou liquide usuel et une 1-(trihalogénométhylsulfényl)-4-aryl-1,2,4-triazolidin-5-one selon la formule (I) de la revendication 1.

7. Procédé de préparation d'un fongicide selon la revendication 6, caractérisé par le fait qu'on mélange un véhicule solide ou liquide usuel avec une 1-(trihalogénométhylsulfényl)-4-aryl-1,2,4-triazolidin-5-one selon la formule (I) de la revendication 1.

8. Procédé de lutte contre les champignons, à l'exception des champignons nuisibles aux humains, caractérisé par le fait qu'on traite les champignons, ou les objets à protéger contre l'attaque de ces champignons, avec une 1-(trihalogénométhylsulfényl)-4-aryl-1,2,4-triazolidin-5-one selon la formule (I) de la revendication 1.

**Revendications pour l'Etat contractant: AT**

1. Fongicide contenant une 1-(trihalogénométhylsulfényl)-4-aryl-1,2,4-triazolidin-5-one de formule (I)

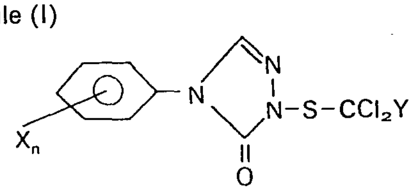

(I)

dans laquelle

X représente hydrogène, halogène, un groupe alkyle ou alcoxy ayant 1 à 5 atomes C, éventuellement substitué par fluor, chlore ou brome, alcényle ayant 2 à 4 atomes C, cyano, nitro, phényle ou phénoxy, le reste phényle ou phénoxy pouvant être substitué par fluor, chlore, brome ou alkyle ayant 1 à 4 atomes C, et

n est un nombre entier de 1 à 5, et

Y représente fluor ou chlore.

2. Fongicide, contenant une 1-(trihalogénométhylsulfényl)-4-aryl-1,2,4-triazolidin-5-one selon la formule générale (I) de la revendication 1.

3. Fongicide selon la revendication 1, caractérisé par le fait que X représente hydrogène, chlore, fluor, trifluorométhyle, méthyle, butyle ou méthoxy, et n est 1 ou 2.

4. Procédé de préparation d'un fongicide selon la revendication 2, caractérisé par le fait qu'on mélange un véhicule solide ou liquide usuel avec une 1-(trihalogénométhyl-sulfényl)-4-aryl-1,2,4-triazolidin-5-one selon la formule (I) de la revendication 1.

5. Procédé de lutte contre les champignons, à l'exception des champignons nuisibles aux humains, caractérisé par le fait qu'on traite les champignons, ou les objets à protéger contre l'attaque de ces champignons, avec une 1-(trihalogénométhylsulfényl)-4-aryl-1,2,4-triazolidin-5-one selon la formule (I) de la revendication 1.

6. Procédé de préparation d'une 1-(trihalogénométhylsulfényl)-4-aryl-1,2,4-triazolidin-5-one selon la formule générale (I) de la revendication 1, caractérisé par le fait qu'on fait réagir, éventuellement en présence d'un solvant, éventuellement avec addition d'une base inorganique ou organique, à des températures comprises

entre −30 et +100° C une 4-aryl-1,2,4-triazolidin-5-one de formule générale (II)

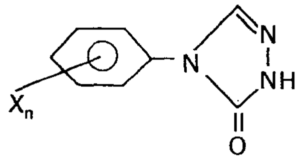

(II)

dans laquelle X et n ont la signification donnée dans la revendication 1, avec un sulfénylchlorure de trihalogénométhyle de formule générale (III)

$$YCCl_2-S-Cl \qquad (III)$$

dans laquelle Y a la signification donnée dans la revendication 1.